# EUROPEAN PATENT SPECIFICATION

(11) **EP 0 407 605 B1**
(45) Date of publication and mention of the grant of the patent: **13.09.1995**
(21) Application number: 90901034.0
(22) Date of filing: 28.12.1989
(51) Int. Cl.: G01N 33/569

(54) **USE OF A REAGENT FOR DETECTING ANTIBODY CORRESPONDING TO ACID-FAST BACTERIUM ANTIGEN**
VERWENDUNG VON EINEM REAGENS ZUM NACHWEIS EINES MIT EINEM SÄUREBESTÄNDIGEN BAKTERIENANTIGEN ÜBEREINSTIMMENDEN ANTIKÖRPERS
UTILISATION D'UN REACTIF POUR LA DETECTION D'UN ANTICORPS CORRESPONDANT A UN ANTIGENE DE BACTERIE RESISTANTE A L'ACIDE

(30) Priority: 18.01.1989 JP 10356/89; 01.04.1989 JP 83836/89
(43) Date of publication of application: 16.01.1991
(73) Proprietor: SAWAI PHARMACEUTICAL CO., LTD., Osaka-shi Osaka 535 (JP); MEDISA SHINYAKU INC., Tokyo 103 (JP)
(72) Inventor: YANO, Ikuya, Osaka 562 (JP); OKA, Shiro, Takaishi-shi Osaka 592 (JP); UENO, Yoshiteru, Toyonaka-shi Osaka 561 (JP); NATSUHARA, Yayoi, Miyakojima-ku Osaka-shi Osaka 534 (JP); YOSHINAGA, Junji, Neyagawa-shi Osaka 572 (JP); KATO, Yoshiko, Nishinomiya-shi Hyogo 662 (JP)
(74) Representative: von Kreisler, Alek, Dipl.-Chem.
(86) International application number: JP8901341
(87) International publication number: WO9008323

(56) References cited:
- JP-A- 5 679 956
- JP-A-57 154 151
- US-A- 4 401 658
- JAPANESE JOURNAL OF BACTERIOLOGY, vol. 44, no. 2, 01 February 1989, JP; Y. OHTSUBO et al., pp. 533-539/
- JOURNAL OF GENERAL MICROBIOLOGY, vol. 134, no. 8, 1988; K. KANEDA et al., pp. 2213-2229/
- JOURNAL OF CLINICAL MICROBIOLOGY, vol. 27, no. 11, 1989; H. IKAWA et al., pp. 2552-2558/

## Description

### TECHNICAL FIELD

The present invention relates to the use of a particular reagent for detecting an antibody against an acid-fast bacterial antigen.

### BACKGROUND ART

The acid-fast bacteria are characterized by the presence of mycolic acid, an extremely high hydrophobic long-chain fatty acid, on the surface layer of cells thereof. The mycolic acid exists as bound to various lipids, saccharides, peptides and other substances as well as arabinogalactan.

Above all, trehalose dimycolate (hereinafter abbreviated as TDM), a mycolic acid bound to saccharide, also known as the cord factor in tubercule bacillus, was once drew much attention as a toxic glycolipid produced by human tubercule bacillus from the viewpoint of its close relationship with the pathogenicity of the bacillus. In recent years, it has been found that TDM is also widely distributed in the cell walls of a large number of other bacterial species of the genus Mycobacterium and nonpathogenic actinomycetes closely related thereto, such as those belonging to the genera Nocardia and Rhodococcus.

It is also known that the mycolic acid found in the cell walls of acid-fast bacteria occurs not only as TDM but also as mycolic acid containing glycolipids (hereinafter abbreviated as MGL) such as glucose-monomycolate, glucose-dimycolate, fructose-monomycolate, arabinose-monomycolate, mannose-monomycolate, trehalose-monomycolate, trehalose-trimycolate and trehalose-tetramycolate.

These MGLs have drawn much attention in that they possess a wide variety of immunopharmacological bioactivities such as immunoadjuvant activities, non-specific protective immunity, granuloma formation capability, macrophage-activating capability and antitumor activities, as well as toxicities [cf. Kekkaku, 63 (3), 41-54 (1988) and the Proceedings of the 11th Meeting of the Japanese Society for Medical Mass Spectrometry, 11, 63-72 (1986)].

A particular kind of mycolic acid is specific to bacterial species, which serve as an important factor for identification of acid-fast bacteria. Meanwhile, there have already been elucidated the subclass composition and molecular species composition of mycolic acid for each bacterial species [cf. the Journal of Clinical Microbiology, 24 (6), 106-1070 (1986) and the Journal of General Microbiology, 134, 2213-2229 (1988)].

It is therefore possible to identify acid-fast bacteria, namely, infecting bacterial species, by detecting the antibody against the mycolic acid derived from each bacterial species or its derivative.

However, since these bacterial species have conventionally been identified mainly by analyzing the morphological and biochemical characters of bacterial cells obtained after isolation culture, this identification method has posed problems such as lack of quickness and unsatisfactory reliability of results due to inconsistent judgement results obtained in some species.

It is a well-known fact that infections with tubercule bacillus or acid-fact bacteria closely related thereto proceed mainly with epithelioid cellular granuloma based on various immunological reactions and cause tubercules and cavitation, the most characteristic lesions. However, there is no method for quick identification of these infectious bacteria. With this background, there is a strong demand for a method of the quick diagnosis of these infections with tubercule bacillus or acid-fast bacteria closely related thereto.

It is an object of the present invention to provide a reagent for identification of acid-fast bacterial species and acid-fast bacterial genera (including the genera Mycobacterium, Nocardia and Rhodococcus).

It is another object of the present invention to provide a method of identification of acid-fast bacterial species and acid-fast bacterial genera (including the genera Mycobacterium, Nocardia and Rhodococcus).

It is yet another object of the present invention to provide a method of quick diagnosis of acid-fast bacterial infections in humans and other animals.

### DISCLOSURE OF THE INVENTION

The present inventors made intensive investigations on mycolic acid and its derivatives, cell wall lipid components of the cells of tubercule bacillus and other acid-fast bacteria, and have found that an antibody produced in response to the administration of MGL obtained from these bacterial cells to living bodies recognizes a mycolic acid moiety (namely, mycolic acid itself or a derivative thereof) as an antigen and also recognizes structural differences among the mycolic acid subclasses. The present inventors have also found that an antibody in acid-fast bacterial infection patients recognizes a mycolic acid, mycolic acid salt, mycolic acid ester or an ester of fatty acid having a carbon number of 14 or more other than mycolic acid with a mono- or disaccharide, as an antigen, and that when using as a sample a body fluid of a patient infected with tubercule bacillus or an acid-fast bacterium closely related thereto, the antibody contained therein recognizes the above-mentioned compounds as antigens. The present inventors made further investigations based on these findings, and completed the present invention.

Accordingly, the present invention provides the use of a compound selected from the group comprising mycolic acids, mycolic acid salts, mycolic acid esters and esters of fatty acids having a carbon number of 14 or more other than mycolic acid with a mono- or disaccharide in an ELISA method for detecting an antibody against an acid-fast bacterial antigen in a serum sample (including the genera Mycobacterium, Nocardia and Rhodococcus). In a prefered embodiment the invention provides a method of diagnosis of acid-fast bacterial infections wherein the difference in absorbance between a sample taken from a healthy human and a sample from a subject is determined by a detection method for an antibody against an acid-fast bacterial antigen described above.

In the present invention, the acid-fast bacterial genera are mainly Mycobacterium, Nocardia and Rhodococcus, etc. Examples of acid-fast bacterial species belonging to the genera Mycobacterium include tubercule bacilli such as M. tuberculosis and M. bovis; and acid-fast bacteria closely related thereto such as M. kansasii, M. leprae, M. intracellulare-M. avium complex and atypical mycobacteria such as M. scrofulaceum and M. fortuitum.

In the scope of the present invention, mycolic acid is defined as a compound having a structure of the following formula (I): 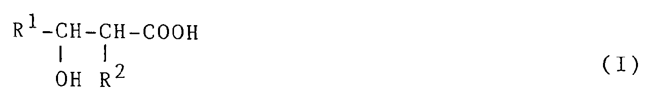
wherein R¹ and R² independently represent a C₁₀₋₆₀ normal or branched aliphatic chain.

Here, R¹ and R² may have groups with different polarities, e.g., cycloalkyl groups such as cyclopropane ring; lower alkyl groups such as methyl group; lower alkoxy groups such as methoxy group; epoxy group, hydroxy group, carbonyl group and carboxyl group, and may have a double bond (preferably 1 to 7 double bonds).

The structure of the mycolic acid obtained from bacterial cells varies widely among the bacterial species and total carbon number, double bond number and the carbon numbers of R¹ and R² are taxonomically significant. As for total carbon number, it varies very widely; for example, C₈₀ or more is often the case with the genus Mycobacterium, C₅₀ or so with the genus Nocardia and C₃₀ or so with the genus Corynebacterium. In addition, R¹ and R² have various groups with different polarities such as cyclopropane ring, methyl group, methoxy group, epoxy group, hydroxy group, carbonyl group and carboxyl group. These facts are already known ["Biseibutu no Kagaku Jikkenho", published by Gakkai Syuppan Center, 131-143 (February 10, 1985, 3rd printing); European Journal of Biochemistry, 139, 173-180 (1984)].

It should be noted, however, that the difference in substituent is significant in identification of bacterial species in the present invention. For example, the following subclasses are known to be obtained from bacterial cells.
(a) α-mycolic acid (C₇₈ <, M. tuberculosis) 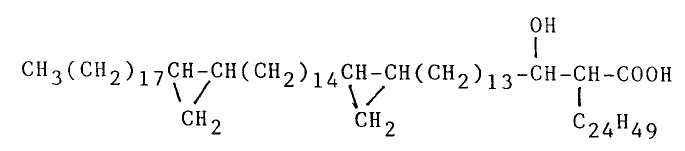
(b) Methoxy-mycolic acid (C₈₅ <, M. tuberculosis) 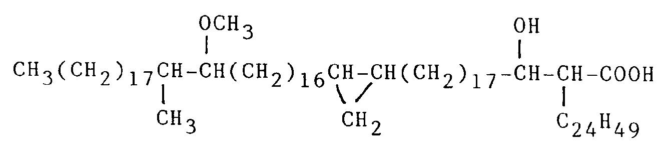
(c) β-mycolic acid (or keto-mycolic acid) (C₈₅ <, M. tuberculosis) 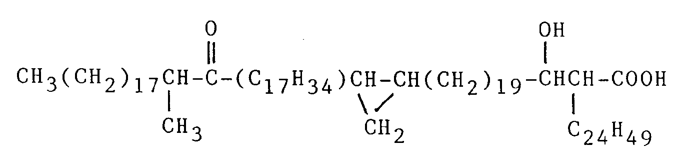
(d) Dicarboxy-mycolic acid (C₆₀ <, M. phlei)

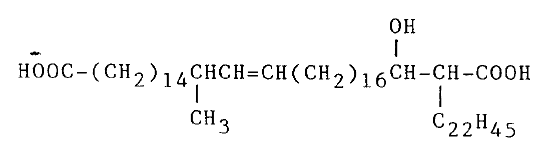

Examples also inclulde α′-mycolic acid (the same as α-mycolic acid except for carbon number) and those having an epoxy group.

The mycolic acid involved in the present invention is defined as above, but the carbon numbers shown above are average values, and the mycolic acid may be a mixture of various mycolic acids with different carbon numbers.

In the present invention, the mycolic acid salt means the salt of the mycolic acid defined above with a metal such as sodium or potassium.

In the present invention, the mycolic acid ester means not only an ester with a mono- or disaccharide but also an alkyl, alkenyl, silyl or aryl ester. These esters are not subject to limitation as long as they do not interfere with the antigen-antibody reaction. Examples of such esters include methyl ester, ethyl ester, t-butyl ester, phenyl ester, trimethylsilyl ester and benzyl ester. The fatty acid having a carbon number of 14 or more (preferably 14 to 80, more preferably 14 to 40) other than mycolic acid means a saturated or unsaturated aliphatic carboxylic acid which may have a substituent such as a hydroxyl group, a C₁₋₆ alkoxy group, a C₁₋₆ alkyl group, an epoxy group, a carbonyl group or a carboxyl group. Examples of simple fatty acids having no substituent include saturated fatty acids such as myristic acid, pentadecanoic acid, palmitic acid, heptadecanoic acid, stearic acid, nonadecanoic acid, arachidic acid, behenic acid, lignoceric acid, cerotic acid, heptacosanoic acid, montanoic acid, melissic acid and vaccenic acid; and unsaturated fatty acids such as oleic acid, elaidic acid, cetoleic acid, erucic acid, brassidic acid, linoleic acid, linolenic acid, arachidonic acid and stearolic acid.

In the present invention, the monosaccharide means not only a normal, branched or cyclic monosaccharide such as pentose, hexose, heptose, octose, nonose or decose but also a substitution product such as deoxy sugar, methyl sugar, thio sugar or amino sugar.

The disaccharide means maltose, cellobiose, gentiobiose, melibiose, lactose, turanose, sophorose, trehalose, isotrehalose, sucrose or isosaccharose.

In the present invention, the alkyl, alkenyl or aryl is not subject to limitation. Examples thereof include alkyls (e.g. normal, branched or cyclic alkyls such as methyl, ethyl, propyl, isopropyl, isobutyl, sec-butyl, tert-butyl, butyl, hexyl, cyclohexyl) which may be substituted; alkenyls (e.g. normal, branched or cyclic alkenyls such as allyl, vinyl, benzyl and styryl); and aryls (e.g. phenyl and naphthyl). Specifically, the C₁₋₆ alkyl in the present invention means an alkyl having a carbon number of 6 or less, such as methyl, ethyl, propyl, isopropyl, isobutyl, sec-butyl, tert-butyl, butyl, n-amyl, isoamyl, hexyl, cyclopentyl and cyclohexyl.

The above-mentioned mycolic acids or mycolic acid esters with a substance other than a saccharide are produced by a method known per se (e.g. extraction and synthesis from bacterial cells).

Extraction from bacterial cells is carried out as follows:

Bacterial cells are subjected to direct hydrolysis and organic solvent extraction, followed by separation by TLC (thin layer chromatography), or glycolipids are extracted and separated from bacterial cells with chloroform-methanol or other solvent, and subjected to hydrolysis, followed by organic solvent extraction of the lipids and TLC separation, to afford the mycolic acids of different subclasses.

These mycolic acids can be further converted to lower alkyl esters by conventional methods. These mycolic acids or mycloic acid esters with a substance other than a saccharide (e.g. mycolic acid C₁₋₆ alkyl esters) can also be obtained by synthesis by conventional methods as follows:

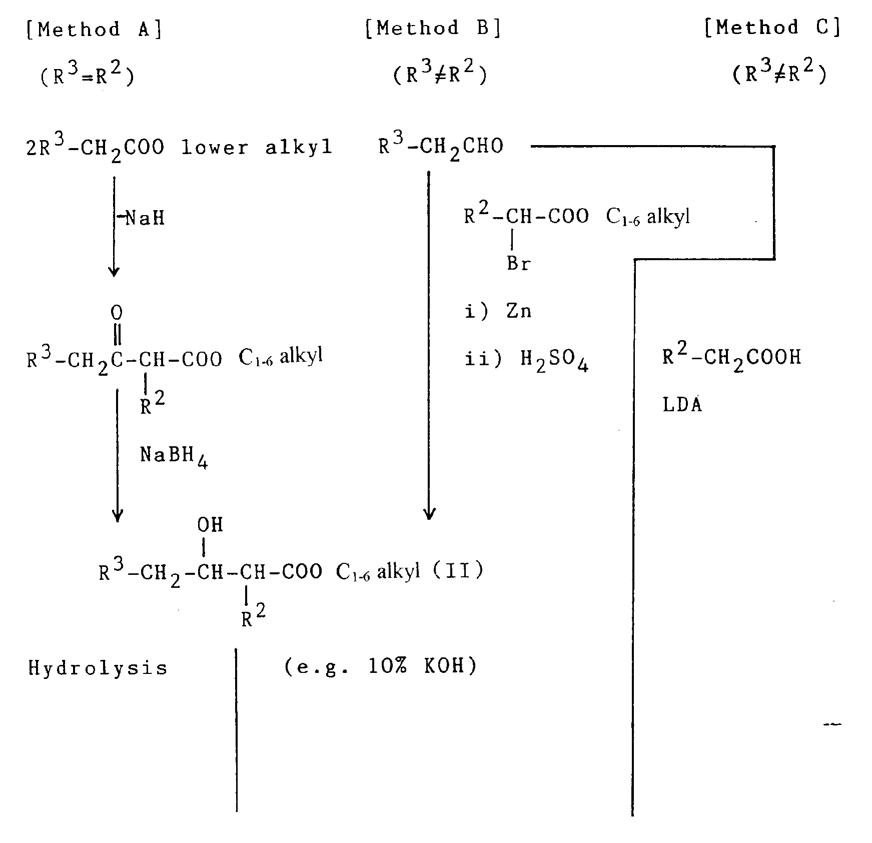

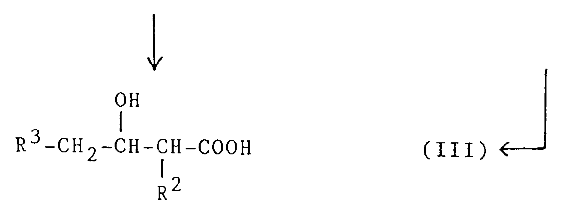

In these formulas, R³-CH₂ is identical with R¹ in the above formula (I) and R² has the same definition as above.

In accordance with Method A, when producing a branched fatty acid like α-alkyl-β-hydroxy fatty acid (III) [the mycolic acid represented by the above formula (I)], the desired product mycolic acid lower alkyl ester (II) can be obtained by Claisen condensation of the fatty acid ester followed by reduction with NaBH₄ as described in literature [Bull. Soc. Chim. Fr., 504-510 (1954)] when R³ and R² represent the same alkyl group.

In accordance with Method B, the mycolic acid C₁₋₆ alkyl ester (II) can be obtained by condensation of an aliphatic aldehyde and an α-bromocarboxylic acid ester by Reformatsky reaction, when R³ and R² are alkyl groups different from each other. Hydrolysis of the compound (II) obtained by Method A or B affords the desired product compound (III) [mycolic acid (I)].

In accordance with Method C, the compound (III) [mycolic acid (I)] can be directly obtained by condensation of an aliphatic aldehyde and a carboxylic acid preferably in the presence of a strong base such as LDA (lithium diisopropylamide) at a concentration of 2 moles or more, when R³ and R² are alkyl groups different from each other.

Synthesis of course affords various mycolic acids with different desired carbon numbers and with uniform composition.

Note that a mycolic acid salt can be prepared easily, for example, by reaction of a mycolic acid with a base.

A fatty acid having a carbon number of 14 or more other than mycolic acid is also prepared by a method known per se.

The mycolic acid (obtained from bacterial cells or by synthesis), mycolic acid salt or fatty acid having a carbon number of 14 or more thus obtained is esterified with a mono- or disaccharide described above by a known method to yield an ester with a saccharide.

In the present invention, the disaccharide is preferably trehalose. In other words, the ester is preferably an ester of mycolic acid with trehalose or another ester of a fatty acid having a carbon number of 14 or more other than mycolic acid with trehalose (hereinafter abbreviated as TFE).

Examples of TFE include trehalose palmitate, trehalose myristate, trehalose stearate, trehalose octadecadienoate, trehalose corynomycolate and trehalose mycolate, specifically, 6,6′-di-O-palmitoyl-α,α-trehalose, 6,6′-di-O-mycoloyl-α,α-trehalose and 2,6′-di-O-oleoyl-α,α-trehalose.

The TFE includes known compounds and is produced by subjecting trehalose and the above-mentioned mycolic acid (obtained from bacterial cells or by synthesis), mycolic acid salt or fatty acid having a carbon number of 14 or more to an esterification process known per se. For example, a di-substitution product of trehalose at the 6 and 6′-positions can be produced by the method described in Japanese Patent Publication Open to Public Inspection No 185599/1983 and Chemistry and Physics of Lipids, vol. 27, pp. 345-352 (1980), or a method analogous thereto.

Also, an asymmetric di-substitution product of trehalose (e.g. substituted at the 2 and 6′-positions) can be produced by protecting each of the 4 and 6-positions and 4′ and 6′-positions of trehalose with a protective group such as tetraisopropyl-disiloxane-1,3-diyl; isomerizing them by a known method; again protecting the 6-position; diacylating the 2 and 6′-positions with a desired fatty acid, and deprotecting the protected groups other than those at the 2 and 6′-positions.

The TFE is preferably an ester comprising trehalose bound with 1 to 4 fatty acids (including mycolic acid) at optional positions, preferably at any of the 2, 2′, 3, 3′, 6 and 6′-positions, with further preference given to an ester comprising trehalose bound with two fatty acids (including mycolic acid) at the 6 and 6′-positions.

In the present invention, it is possible to even identify infecting bacterial species when a mycolic acid ester of each subclass is used as a reagent for the detection of the antibody against an acid-fast bacterial antigen; therefore, the trehalose mycolic acid ester (hereinafter abbreviated as TME) is preferable among the TFEs.

Examples of the TME include trehalose monomycolate, trehalose dimycolate, trehalose trimycolate and trehalose tetramycolate, with preference given to trehalose-6,6′-dimycolate.

Specific examples of the TME include 6′-O-corynomycoloyl-2-O-pentadecanoyl-α,α-trehalose, 2-O-icosanoyl-6′-O-(3-hydroxy-2-n-octadecyl -tetracosanoyl)-α,α-trehalose, 2-O-(9-octadecenoyl)-6′-O-(3-hydroxy-2-tetradecyl -11-icosenoyl)-α,α-trehalose, 2-O-pentadecanoyl-3-O-octadecanoyl-6′-O-(3-hydroxy-2-n-tetradecyl -docosanoyl)-α,α-trehalose, 2,3-di-O-eicosanoyl-6′-O-(3-hydroxy-2-n-octadecyl -tetracosanoyl)-α,α-trehalose and 2,3-di-O-(9-octadecenoyl)-6′-O-(3-hydroxy-2-tetradecyl -11-icosenoyl)-α,α-trehalose.

The TME includes known compounds, and can be extracted and isolated from acid-fast bacteria such as those of the genera Mycobacterium, Nocardia, Rhodococcus and Corynebacterium by a known method as in the method of the extraction of mycolic acid from bacterial cells described above. In this case, it is possible to obtain trehalose-6-monomycolate, trehalose-6,6′-dimycolate (cord factor), trehalose-2,3,6′-trimycolate, trehalose-2,6,6′-trimycolate and others derived from various bacterial species.

As stated above, it is possible to separate TME directly from bacterial cells on the basis of the number of mycolic acids bound to trehalose but not possible on the basis of the kind (subclass) such as different mycolic acid substituent. etc. For this reason, when a desired mycolic acid derived from bacterial cells is bound to a particular position of trehalose, TME can be produced by extracting, separating and isolating mycolic acid alone from an acid-fast bacterium by a known method and subjecting them and trehalose to the esterification process known per se which has been described above.

In the present invention, it is also possible as in the case of disaccharides, to produce an ester of a mycolic acid (produced from bacterial cells or by synthesis), a mycolic acid salt or a fatty acid having a carbon number of 14 or more with a monosaccharide.

In the detection of the antibody against the acid-fast bacterial antigen, identification of acid-fast bacterial species, identification of acid-fast bacterial genera (including Mycobacterium, Nocardia and Rhodococcus) or diagnosis of acid-fast bacterial infections using the reagent of the present invention, it is preferable that the mycolic acid, mycolic acid salt, mycolic acid ester (hereinafter abbreviated as MS) or the ester of fatty acid having a carbon number of 14 or more other than mycolic acid with a mono- or disaccharide (hereinafter abbreviated as SFE) be used after being immobilized on a plate. These antigens are then brought into contact with an antibody-containing sample (e.g. body fluid, particularly blood, serum, plasma, pleural effusion, ascites fluid or urine of a patient infected with tubercule bacillus) and analyzed by the ELISA (enzyme-linked immunosorbent assay) method, a method known per se.

Thus, two methods are available for the detection; the one-step assay method and the two-step assay method. In the one-step assay method, a solid phase prepared by immobilizing various kinds of MS or SFE onto wells, a sample, and an enzyme-labeled antibody are reacted at the same time. After a given length of time, the unreacted portion of the labeled antibody is removed by washing, followed by determination of the enzyme activity of the solid phase to identify the antibody contained in the sample according to the MS or SFE.

In the two-step assay method, an immobilized antigen and a sample are reacted. After a given length of time, the unreacted portion of the antibody in the sample is removed by washing, followed by reaction with an enzyme-labeled antiantibody. After a given length of time, the unreacted portion of the labeled antibody is removed by washing, followed by determination of the enzyme activity of the solid phase to identify the antibody contained in the sample according to the MS or SFE. For further identification in more detail, that is, identification of acid-fast bacterial species, MS separated for each subclass is immobilized onto wells.

The method of immobilization is not subject to limitation as long as the object of the present invention is not interfered with. For example, MS or SFE is dissolved in an organic solvent such as hexane, isopropanol or ethanol, and the resulting solution is added to wells of a microplate, etc. to immobilize the MS or SFE. Immobilization is achieved in about 2 to 48 hours. It is preferable to wash the wells with an appropriate solvent and block it after immobilization.

The reaction of MS or SFE in the wells and the antibody in the sample is carried out under the condition in which the reaction proceeds easily (e.g. in a humid box at a temperature of 20 to 40°C and a humidity of 70 to 100%) for about 30 minutes to 2 hours until it completes itself. After completion of the reaction, an antiantibody is added with or without removal of the unreacted substances in the wells and the reaction is carried out in the state in which the reaction proceeds easily (e.g. in a humid box at a temperature of 20 to 40°C and a humidity of 70 to 100%) for about 10 minutes to 2 hours until it completes itself. The reaction residue is removed and the wells are washed with an organic solvent such as hexane or isopropanol, a phosphate buffer or another washing agent.

The above-mentioned antiantibody is an antiantibody against the above-mentioned primary antibody and is prepared using an animal (no limitation is posed on the animal species; examples of usable animals include goats, horses, sheep and bovines). It is preferable to use an antiantibody against IgM (IgG may be included) when using a mycolic acid, mycolic acid salt or mycolic acid ester of each subclass to identify bacterial species, and to use an antiantibody against IgM + IgG when using an ester of a fatty acid having a carbon number of 14 or more other than mycolic acid with a saccharide (mono- or disaccharide) to identify bacterial genera. In the present invention, an antiantibody coupled with a marker is used. Here, any substance normally used for immunoassay can be used as a marker, and examples of such substances include enzymes, radioactive substances, luminescent substances and fluorescent substances. Examples of enzymes include peroxidase, β,D-galactosidase and alkaline phosphatase. Examples of radioactive substances include iodine and deuterium. Examples of luminescent substances include acrydium salts. Examples of fluorescent substances include fluorescein isothiocyanate.

The purpose of producing an antiantibody coupled with a marker can be fully accomplished by a method known per se.

Then, the antiantibody involved in the reaction is assayed as follows: When the antiantibody is coupled with, for example, peroxidase as a marker, the peroxidase is reacted with o-phenylenediamine and hydrogen peroxide to cause color development, followed by optical density (OD) determination at a main wavelength of 492 nm and a subwavelength of 690 nm.

It is desirable that the results be calculated as differences in optical density difference (ODD) between the average OD of a healthy human as control and the OD of the sample. When ODD is not less than 0.100, it indicates the presence of a significant level of an antibody produced by an acid-fast bacterium, and thus the subject from which the sample was collected (including non-human animals) is judged as being infected with an acid-fast bacterium (tubercule bacillus or an acid-fast bacterium closely related thereto).

The samples are body fluids of animals including humans. Examples of non-human animals include mammals such as horses, swines and chickens, and birds. Examples of usable body fluids include serum, plasma, cerebrospinal fluid, saliva, pleural effusion, ascites fluid and urine. In the present invention, the sample can be prepared in accordance with a known method. When assaying the antibody in, for example, serum, it is preferable to use the serum in dilution at 40 to 640 folds, ideally 160 folds.

The reagent for the detection of the antibody against an acid-fast bacterial antigen of the present invention, the method of the detection of the antibody against an acid-fast bacterial antigen using said reagent, and the method of the diagnosis of acid-fast bacterial infections based on said detection method are simpler in procedures and offer much higher specificity, in comparison with conventional reagents and methods.

Particularly, the reagent of the present invention allows very quick antibody detection, bacterial identification and infection diagnosis since an assay is conducted by directly using a body fluid of patients with an acid-fast bacterial infection as a sample based on the ELISA method.

Furthermore, since reagents used for the present invention (MS and SFE) can also be produced by synthesis, they can be easily supplied in large amounts on an industrial scale.

In addition, the reagent and the method of the present invention permit high sensitive assay, enabling the assay of a trace amount of antibody present in serum.

Thus, simple and quick detection of the antibody against an acid-fast bacterial antigen can be conducted using the reagent by the method of the present invention and in turn identification of acid-fast bacterial genera (including the genera Mycobacterium, Nocardia and Rhodococcus) can be performed. According to the present invention, a diagnosis to identify the infecting acid-fast bacterium is made on the basis of acid-fast bacterial species identification using a mycolic acid, mycolic acid salt or mycolic acid ester (MS) of each subclass as a reagent for the detection of an antibody against an acid-fast bacterial antigen, thereby resulting in particular effect that quick choice of therapeutic drug for the disease is possible.

### BRIEF DESCRIPTION OF THE DRAWINGS

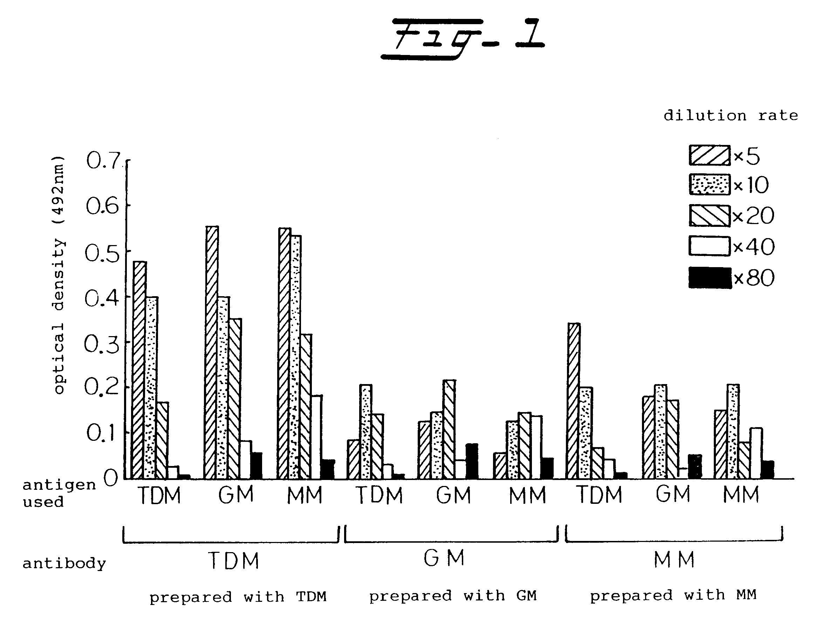

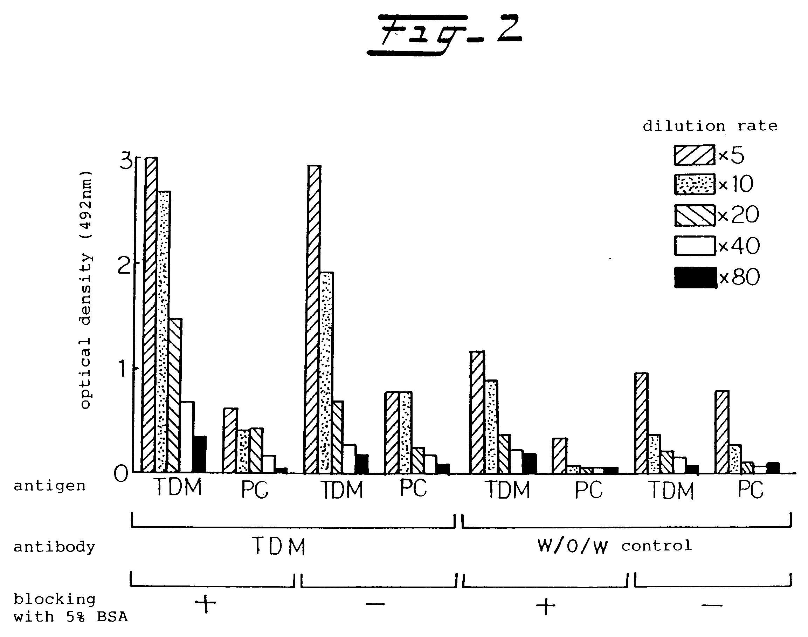

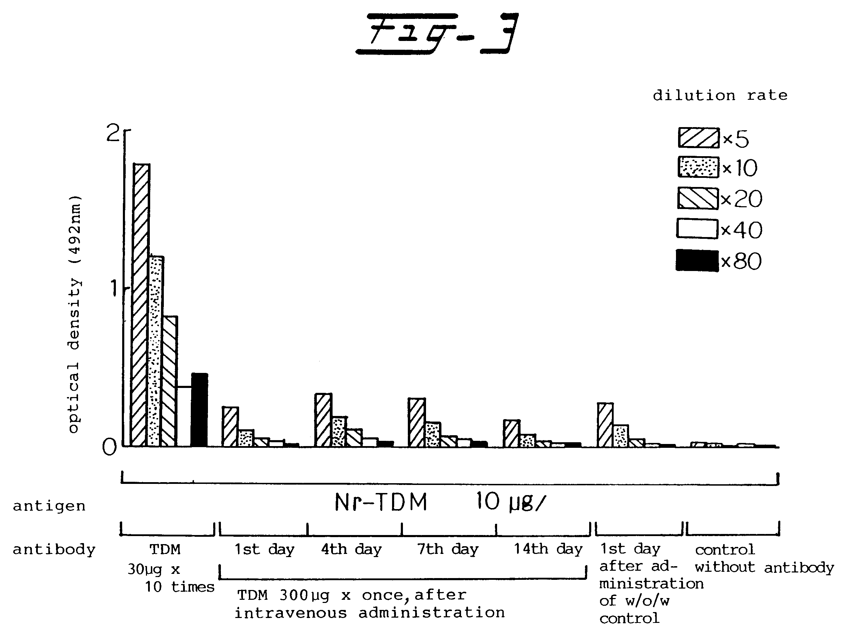

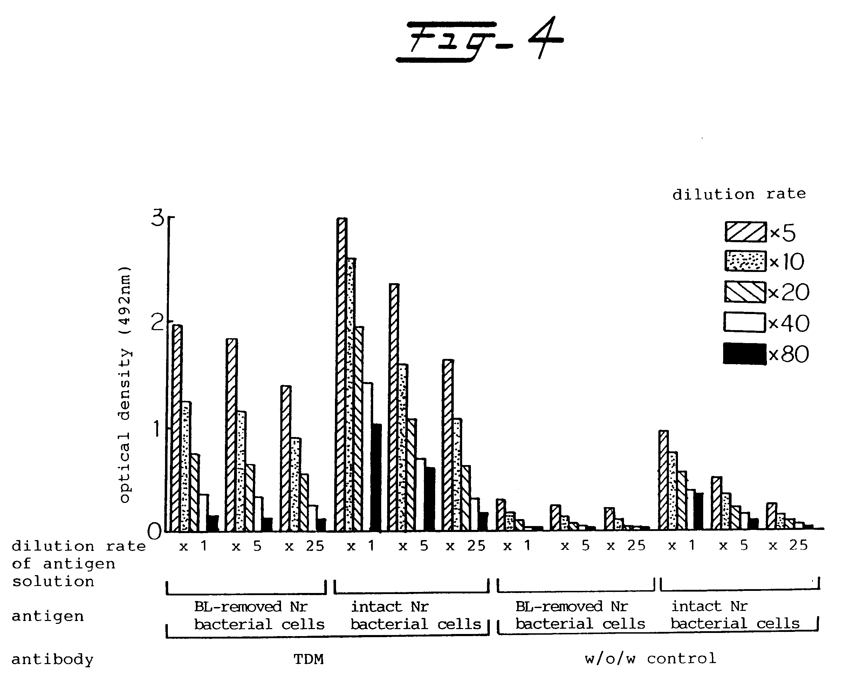

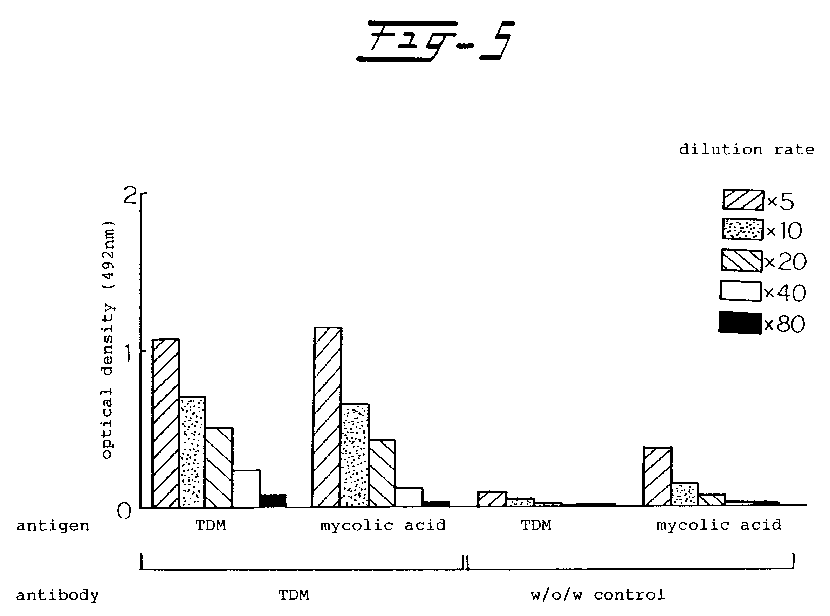

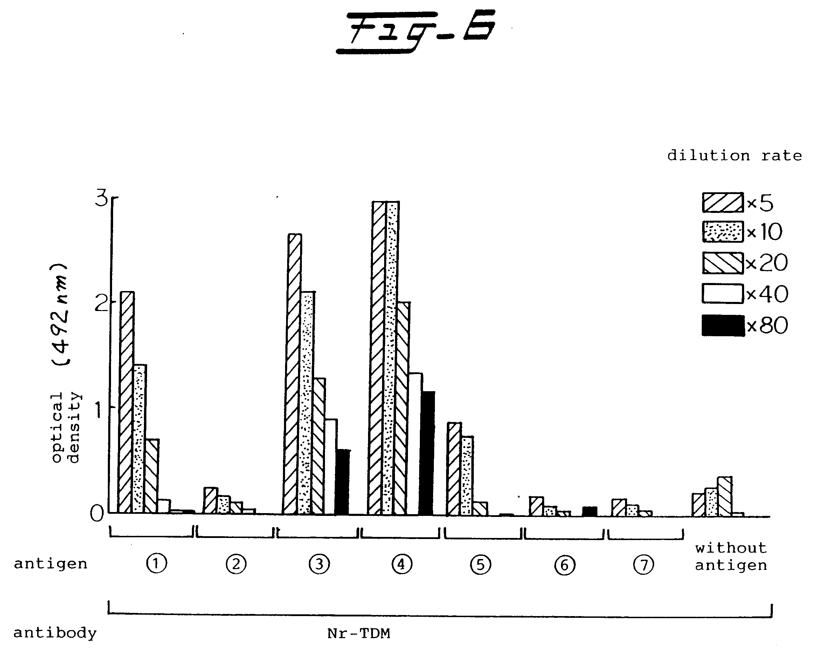

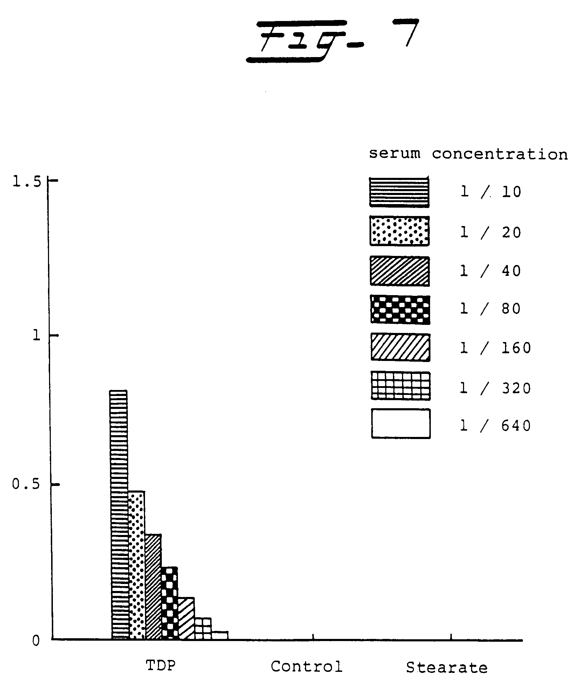

Fig. 1 is a graph showing the detection of the antibodies against trehalose-dimycolate (TDM), glucose-monomycolate (GM) and mannose-monomycolate (MM), all derived from Nocardia rubra (Nr-) by the ELISA method. Fig. 2 is a graph showing the detection of the antibody against Nr-TDM by the ELISA method. Fig. 3 is a graph showing differences in antibody titer against Nr-TDM determined by the mouse immunization method. Fig. 4 is a graph showing the reactions using as antigens Nr bacterial cells with and without the mycolic acid removal. Fig. 5 is a graph showing the antigenicities of mycolic acid methyl esters. Fig. 6 is a graph showing the reactivities obtained using as antigen various TDMs and mycolic acid methyl esters. Fig. 7 is a graph showing the detection of antibody by the ELISA method with trehalose-6,6′-dipalmitate (TDP) as an antigen.

### BEST MODE OF CARRYING OUT THE INVENTION

The present invention is hereinafter described in more detail by means of the following examples, but the invention is not by any means limited by them.

Note that in Examples 1 through 10, assays were made by the ELISA procedure described below, with serum dilution rates of 5, 10, 20, 40 and 80 folds in Examples 1 through 6. The results are shown in respective figures.

### [Assay by ELISA]

An ethanol or isopropanol solution of the antigen is dispensed to a 96-well microplate at 50 µl/well. This microplate is then kept standing overnight to evaporate the solution to dryness to adsorb the antigen.

To this microplate is added 100 µl/well of a phosphate buffered saline (pH 7.4) supplemented with 0.5% Tween® 20 (hereinafter abbreviated as PBS-T), and the microplate is kept standing at room temperature for 10 minutes. Otherwise, 100 µl/well of PBS-T supplemented with 5% bovine serum albumin is added, and the microplate is kept standing at room temperature for 2 hours.

After the PBS-T or the PBS-T supplemented with 5% bovine serum albumin is removed by suction, 50 µl/well of the antibody (serial dilution of serum) is dispensed, and the microplate is kept standing at room temperature for 1 hour.

After 3 times of washing with PBS-T, 50 µg/well of peroxidase-labeled antimouse Ig(G+M) was added, and the microplate is kept standing at room temperature for 1 hour.

After 3 times of washing with PBS-T, 50 µl/well of citrate buffer supplemented with both o-phenylenediamine and aqueous hydrogen peroxide (pH 4.9) is dispensed, followed by addition of 50 µl/well of 6 N HCl and determination of optical density at 492 nm using a microplate reader.

### Example 1

### Detection of antibodies against Nr-TDM, GM and MM by the ELISA method

Antigens: Trehalose-dimycolate (hereinafter abbreviated as TDM), glucose-monomycolate (hereinafter abbreviated as GM) and mannose-monomycolate (hereinafter abbreviated as MM), all derived from Nocardia rubra (hereinafter abbreviated as Nr-), were each prepared as a 200 µg/ml ethanol solution. Each solution was dispensed to a 96-well microplate at 10 µg/50 µl/well, and the microplate was kept standing overnight to evaporate the solution to dryness for antigen adsorption.

The results for these antigens are respectively represented by TDM, GM and MM in Fig. 1.

Antibodies: ICR mice were dosed with Nr-derived (Nr-) TDM, GM or MM in the form of a water-in-oil-in-water type emulsion (hereinafter abbreviated as w/o/w emulsion) containing 3.2% Freund's incomplete adjuvant at a dose of 30 µg/mouse by intravenous injection via tail vein (hereinafter abbreviated as i.v.) 10 times every other day. Two days after the final administration, blood was collected. The serum thus obtained was 5-fold diluted with PBS-T, after which it was thermally treated at 56°C for 30 minutes to inactivate the complements in the serum (hereinafter referred to as inactivation) to yield an original solution, which was then used in serial dilutions with PBS-T. The results for these antibodies are respectively represented by TDM, GM and MM in Fig. 1.

### (Results)

As shown in Fig. 1, the following results were obtained:
1. An antibody which recognizes MGL as an antigen was detected in the serum of mice dosed with MGL (mycolic acid-containing glycolipid) 30 µg x 10 times i.v..
2. The serum of TDM-administered mice showed high antibody titer against all of TDM, GM and MM.

### Example 2

### (Purposes)

1. To examine non-specific adsorption of mouse serum globulin by the antigen Nr-TDM adsorbed to a plate, a serum from the w/o/w control group (described later) was used as an antibody.
2. To determine whether or not the reaction of the antigen glycolipid with mouse serum immunoglobulin is against a non-specific lipid, egg yolk phosphatidylcholine (PC) was used as an antigen.
3. The effects of blocking procedure using 5% bovine serum albumin (BSA) to the antigen-adsorbed plate were evaluated.

Antigens: Nr-TDM or PC was prepared as a 200 µg/ml ethanol solution. Each solution was dispensed to a 96-well microplate at 10 µg/50 µl/well. This microplate was kept standing overnight to evaporate the solution to dryness for antigen adsorption.

The results for these antigens are respectively represented by TDM and PC in Fig. 2.

Antibodies:
1) 5-fold dilution of a serum obtained from ICR mice dosed with Nr-TDM 30 µg x 10 times (the same as in Example 1).
2) Inactivated 5-fold dilution with PBS-T of a serum obtained by blood sampling from ICR mice dosed with 0.2 ml/mouse of a w/o/w emulsion containing no glycolipid 10 times every other day. The results for these antibodies are respectively represented by TDM and w/o/w control in Fig. 2.

### (Results)

As shown in Fig. 2, the following results were obtained:
1. Almost no reaction took place between the antigen (TDM or PC) and the serum of the w/o/w control group; it was confirmed that the adsorption was not non-specific.
2. No reaction took place between the antigen PC and the antibody of the TDM group; it was confirmed that the reaction was not a reaction with a non-specific lipid.
3. The blocking procedure with 5% BSA had no particular effects

### Example 3

### Examination of differences in antibody titer against Nr-TDM by the mouse immunization method

Antigen: Nr-TDM was prepared into a 200 µg/ml ethanol solution. This solution was dispensed to a 96-well microplate at 10 µg/50 µl/well, followed by antigen adsorption.

Antibodies:
1) 5-Fold dilution of a serum obtained from ICR mice dosed with Nr-TDM 30 µg x 10 times (the same as in Example 1).
2) 5-Fold dilutions of sera obtained by blood sampling from ICR mice at 1, 4, 7 and 14 days following administration of Nr-TDM 300 µg x 1 time.
3) 5-Fold dilution of a serum obtained from ICR mice 1 day after administration of 02 ml/i.v. of a w/o/w emulsion containing no glycolipid.

### (Results)

As shown in Fig. 3, the following results were obtained:
1. The same absorbance at 492 nm as in the w/o/w control group was obtained at 1 through 14 days following administration of Nr-TDM 300 µg x 1 time; no antibody was detected.
2. Almost no fluorescent coloring occurred in the wells not supplemented with mouse serum; it was confirmed that Nr-TDM showed no non-specific adsorption of the secondary antibody [peroxidase-labeled antimouse Ig(G+M)].

### Example 4

### Examination of reactions when using as antigens Nr bacterial cells with and without mycolic acid removal

Antigens:
1) Nr bacterial cells.
2) Nr bacterial cells were mixed with a 2:1 (v/v) mixture of chloroform and methanol, followed by ultrasonication to remove the extractable lipid and then acid hydrolysis (see Note 1) to remove the bound lipid (BL).

These antigens 1) and 2) were each suspended in a borate buffer so that the absorbance at 525 nm became 0.43. Each original suspension thus obtained was diluted at 1/1, 1/5 and 1/25 fold dilution rates. Each dilution thus obtained was dispensed to a plate at 50 µl/well. This plate was kept standing overnight for antigen adsorption, followed by supernatant removal by suction.

Antibodies:
1) Serum collected from ICR mice dosed with Nr-TDM 30 µg x 10 times (the same as in Example 1).
2) Serum collected from ICR mice dosed with w/o/w control 0.2 ml x 10 times (the same as in Example 2).

### (Results)

As shown in Fig. 4, the following results were obtained:
1. The Nr bacterial cells reacted with the antibody.
2. The reactivity to the antibody decreased in response to removal of the lipid (mycolic acid) from the Nr bacterial cells.

(Note 1): Acid hydrolysis: After addition of 6 N HCl in an amount of about one-third of the cell suspension, the cell suspension was heated at 90°C overnight, followed by lipid extraction with n-hexane. The resulting water layer was used as antigen.

### Example 5

### Examination of the antigenicity of mycolic acid lower alkyl ester

Antigens:
1) Nr-TDM.
2) Mycolic acid methyl ester obtained by hydrolysis and methylation of Nr-TDM.

These antigens 1) and 2) were each prepared into a 200 µg/ml ethanol solution. Each solution was dispensed to a 96-well microplate at 10 µg/50 µl/well, followed by antigen adsorption.

Antibodies:
1) Serum of ICR mice dosed with Nr-TDM 30 µg x 10 times (the same as in Example 1).
2) Serum of ICR mice dosed with w/o/w control 0.2 ml x 10 times (the same as in Example 2).

### (Results)

As shown in Fig. 5, the following results were obtained:

The mycolic acid lower alkyl ester showed an antigenicity equivalent to that of TDM.

### Example 6

Antigens:
1) Nr-TDM.
2) M. intracellulare [hereiniafter referred to as M. int.]-TDM.
3) M. kansaii [hereinafter referred to as M. kan.]-TDM.
4) Nr-derived mycolic acid methyl ester.
5) M. int.-derived α-mycolic acid (M₁) methyl ester.
6) M. int.-derived keto-mycolic acid (M₂) methyl ester.
7) M. int.-derived dicarboxy-mycolic acid (M₃) methyl ester.

Each antigen was prepared into a 200 µg/ml ethanol solution. Each solution was dispensed to a 96-well microplate at 10 µg/50 µl/well. The microplate was kept standing overnight to evaporate the solution to dryness for antigen adsorption.

The results for these antigens are respectively represented by 1) through 7) in Fig. 6.

Antibody: 1) Serum of ICR mice dosed with Nr-TDM 30 µg x 10 times (the same as in Example 1).

### (Reference)

1. All Nr-derived mycolic acids are α-mycolic acids having a total carbon chain length of C₃₆₋₄₈ (average 44) and a relatively short side chain of C₁₀₋₁₄ at the α-position.
2. M. int. has three kinds of mycolic acid which respectively have an average carbon chain length as follows:
   - M₂: α-mycolic acid: C₈₀
   - M₂: Keto-mycolic acid: C₈₅
   - M₃: Dicarboxy-mycolic acid: C₆₉
3. M. kan. has three kinds of mycolic acid, with the predominance of α-mycolic acid. The average carbon chain length is as follows:
   - α-mycolic acid: C₈₀
   - Methoxy-mycolic acid: C₇₈
   - Keto-mycolic acid: C₈₃

### (Results)

As shown in Fig. 6, the following results were obtained:
1. The antibody against Nr-TDM reacted with the Nr-TDM, Nr-derived mycolic acid (α-mycolic acid) methyl ester, M. kan.-TDM (most percentage of the mycolic acid is α-mycolic acid) and M. int.-derived a-mycolic acid (M₁) methyl ester, but did not react with the M. int.-derived keto-mycolic acid (M₂) methyl ester or M. int.-derived dicarboxy-mycolic acid (M₃) methyl ester.
2. M. kan.-derived and M. int.-derived mycolic acid methyl esters were almost equivalent to each other in carbon chain length, but were different from each other in subclass composition.
3. Based on these findings, the antiserum of mice immunized with Nr-TDM is considered highly specific to Nr-derived α-mycolic acid esters.

### Example 7

Nr-TDM was subjected to acid hydrolysis in accordance with the methods of Examples 4 and 5 to yield a vinyl ester and phenyl ester thereof, which were used as antigens.

Antigens:
1) Nr-derived mycolic acid vinyl ester.
2) Nr-derived mycolic acid phenyl ester.
3) α-mycolic acid (synthesized product).
4) α-mycolic acid methyl ester (synthesized product).

Each antigen was prepared into a 200 µg/ml ethanol solution. Each solution was dispensed to a 96-well microplate at 10 µg/50 µl/well. This microplate was kept standing overnight to evaporate the solution to dryness for antigen adsorption.

Antibody: Serum of mice dosed with Nr-TDM 30 µg x 10 times (the same as in Example 1).

### (Results)

1. The antibody against Nr-TDM reacted with the Nr-derived mycolic acid (α-mycolic acid) vinyl ester and phenyl ester, and also with the α-mycolic acid prepared by organic synthesis and its methyl ester.
2. As stated above, the antiserum collected from mice immunized with Nr-TDM is considered highly specific not only to Nr-derived but also to synthesized α-mycolic acid and its esters.

### Example 8

M. int.-TDM was subjected to acid hydrolysis in accordance with the method of Example 4, followed by isolation and purification of M₂ and M₃. A glucose ester of M₂ and an arabinose ester of M₃ were each prepared and used as antigens.

Antigens:
1) M. int.-derived keto-mycolic acid (M₂) glucose ester.
2) M. int.-derived dicarboxy-mycolic acid (M₃) arabinose ester.
3) Nr-TDM.
4) M. kan.-TDM.

Each antigen was prepared in a 200 µg/ml ethanol solution. Each solution was dispensed to a 96-well microplate at 10 µg/50 µl/well. This microplate was kept standing overnight to evaporate the solution to dryness for antigen adsorption.

Antibody: Serum of mice dosed with M. int.-TDM 30 µg x 10 times (prepared in the same manner as in Example 1).

### (Results)

1. The antibody against M. int.-TDM reacted with the M₂ glucose ester and M₃ arabinose ester but showed a lower reactivity with Nr-TDM and M. kan.-TDM.
2. Based on these findings, it is considered that the antiserum of mice immunized with M. int.-TDM shows high specificity to keto-mycolic acid ester and dicarboxyl-mycolic acid ester, and its reaction response depends not on saccharides but on fatty acids (keto- and dicarboxy-mycolic acids).

### Example 9

Antigens:
1) Trehalose-6,6′-dipalmitate (hereinafter abbreviated as TDP).
2) Hexane (control).
3) Methyl stearate (comparison).

Each antigen was prepared into a 200 µg/ml ethanol solution. Each solution was dispensed to a 96-well microplate at 10 µg/50 µl/well. This microplate was kept standing overnight to evaporate the solution to dryness for antigen adsorption. The results of detection obtained from these antigens are respectively represented by TDP, Control and Stearate in Fig. 7.

Antibody: A serum collected by blood sampling from patients infected with tubercule bacillus who tested positive in bacterial detection was 5-fold diluted with PBS-T and inactivated (heat treated at 56°C for 30 minutes) to yield an original solution, which was then used in serial dilutions with PBS-T.

### (Results)

As shown in Fig. 7, the following results were obtained:
1. The serum of patients infected with tubercule bacillus specifically recognized TFE as an antigen.
2. The serum of patients infected with tubercule bacillus showed a high antibody titer against TFE as well.

Note that when a serum obtained by blood sampling from a healthy human was assayed by the ELISA method in the same manner as above, no color development occurred.

### Example 10

The procedure of Example 9 was followed except that TDP was changed to the compounds shown below.
1) M. tuberculosis-derived GM and MM (α- , methoxy- or keto-mycolic acids).
2) M. int.-derived α-mycolic acid (M₁) or keto-mycolic acid (M₂) methyl, vinyl or phenyl ester.
3) M. kan.-derived TDM, GM and MM (α-, methoxy- or keto-mycolic acids).
4) 6-O-palmitoyl-glucose (synthetic monoester).
5) 6-O-palmitoyl-mannose (synthetic monoester).
6) 6,6′-di-O-corynomycoloyl-α,α-trehalose [synthetic TFE (diester)].
7) 6,6′-di-O-palmitoyl-α,α-trehalose [synthetic TFE (diester)].
8) 6,6′-di-O-mycoloyl-α,α-trehalose [synthetic TFE (diester)].
9) 2,6′-di-O-oleoyl-α,α-trehalose [synthetic TFE (diester)].
10) 6′-O-corynomycoloyl-2-O-pentadecanoyl-α,α-trehalose [synthetic TFE (diester)].
11) 2-O-icosanoyl-6′-O-(3-hydroxy-2-n-octadecyl -tetracosanoyl)-α,α-trehalose [synthetic TFE (diester)].
12) 2-O-(9-octadecenoyl)-6′-O-(3-hydroxy-2-tetradecyl -11-icosenoyl)-α,α-trehalose [synthetic TFE (diester)].
13) 2-O-pentadecanoyl-3-O-octadecanoyl-6′-O-(3-hydroxy-2-n-tetradecyl -docosanoym)-α,α-trehalose [synthetic TFE (triester)].
14) 2,3-di-O-eicosanoyl-6′-O-(3-hydroxy-2-n-octadecyltetracosanoyl)-α,α-trehalose [synthetic TFE (triester)].
15) 2,3-di-O-(9-octadecenoyl)-6′-O-(3-hydroxy-2-tetradecyl-11-icosenoyl)-α,α-trehalose [synthetic TFE (triester)].

### (Results)

The serum of patients infected with tubercule bacillus specifically recognized not only TFE but also esters of fatty acids other than mycolic acid with a mono- or disaccharide and mycolic acid esters.

### Example 11

### Method of identifying the genus Mycobacterium and method of diagnosis of acid-fast bacterial infections using samples collected from patients infected with acid-fast bacteria and healthy humans

### (Preparation of glycolipid from bacterial cells)

M. tuberculosis H37Rv. strain was used as the starting material for cord factor antigen.

### (Isolation and purification of cord factor antigen)

Bacterial cells were suspended in a mixture of chloroform and methanol (2:1, v/v), followed by cell disruption by ultrasonication. The extract from the chloroform layer was evaporated to dryness and dissolved in a small amount of a mixture of chloroform and methanol (2:1, v/v). These extracts were subjected to silica gel TLC using chloroform-methanol-acetone-acetic acid (90:10:6:1, v/v) as a developing solvent. Several bands appeared, out of which trehalose- 6,6′-dimycolate (TDM) was separated and recovered using a mixture of chloroform and methanol (2:1, v/v).

### (Samples)

Sera collected from 50 patients infected with an acid-fast bacterium and 55 healthy humans were used as samples.

Healthy humans: All but one were positive in tuberculin dermal reaction.

Patients: All patients were confirmed bacteriologically positive in an acid-fast bacteria detection test by both smear examination and culture. A niacin test revealed that, of the 50 patients, 45 were patients of M. tuberculosis (tuberculosis) infection and the other 5 were patients of atypical mycobacterial infection. All patients had a pulmonary disease, of whom 1 had a complication of pleurisy and 7 had a complication of diabetes mellitus. The patients were aged in the range of 20 to 80. The blood samples were collected after special chemotherapy in 46 patients and before the treatment in 4 patients.

Also separately tested were sera of patients not infected with acid-fast bacterium and having a cancer (adenocarcinoma) in one lung.

### (Assay by the ELISA method)

An ELISA test was conducted on a polystyrene microtiter plate (Falcon 3915, produced by Becton Dickson Co.). The purified cord factor (TDM) antigen was dissolved in n-hexane to reach a concentration of 0.1 mg/ml. A 50 µl portion of this sample was added to each well (TDM 5 µg/well).

After plate drying at room temperature, 150 µl of an immunization blocking solution containing PBS-T was added to each well, and the plate was kept standing at room temperature for 1 hour in an incubator, followed by removal by suction. Then, 50 µg/well of a 160-fold serum dilution was added, and the plate was kept standing for 1 hour. Peroxidase-labeled goat antihuman IgG (500-fold dilution with PBS-T) was used as a secondary antibody (antiantibody). After the plate was kept standing for 1 more hour in an incubator, a substrate solution diluted so that 1 mg/ml of o-phenylenediamine was contained in a solution containing 0.1 M citric acid, 0.2 M Na₂HPO₄:12H₂O and 0.04% aqueous hydrogen peroxide was added.

The reaction was terminated by the addition of 6 N HCl, and absorbances at 492-630 nm were determined using a microplate reader. Each incubator was maintained at room temperature, and the plate was washed thrice with PBS-T during each reaction.

The experimental results were evaluated by ODD.

### (Results)

Healthy humans: The average OD was 0.099, based on which ODD was calculated. The OD in the healthy humans ranged from 0.096 to 0.102, and none of them were judged to be positive.

Patients: Of the 50 acid-fast bacterial infection patients, 48 (43 of the 45 tubercule bacillus infection patients and all of the 5 atypical mycobacterial infection patients) were judged to be positive (ODD exceeding 0.100) (96% sensitivity).

Note that the average ODD in the tuberculosis patients was 1.273, and the average ODD in the atypical mycobacterial infection patients who were negative in the niacin test was 1.356. The ODD of the patients not infected with acid-fast bacteria having a cancer in one lung was 0.009, and they were judged to be negative.

None of the 55 healthy humans were judged to be positive (ODD exceeding 0.100).

These findings show that the antibody which reacts with the M. tuberculosis-derived cord factor (TDM) occurred in sera of patients positive in the acid-fast bacterial detection test, and was not detected in healthy humans (specificity 100%).

### Example 12

Using sera of 29 tuberculosis patients judged to be positive in Example 11, the procedure of Example 11 was followed except that the cord factor (TDM) used in Example 11 was replaced by the M. kan., M. int. or Nr-derived cord factor or trehalose-6,6′-dicorynomycolate (synthesized product). All these patients were judged to be positive to all the 4 antigens.

### Example 13

The procedure of Example 12 was followed using trehalose-6-monomycolates obtained from the bacterial cells described in Example 12. All samples were judged to be positive.

### Example 14

The procedure of Example 12 was followed except that the dilution rate of the sera of 29 tuberculosis patients judged to be positive in Example 11 was changed to 1280 folds. All samples were judged to be positive.

## Claims

1. Use of a compound selected from the group comprising mycolic acids, mycolic acid salts, mycolic acid esters and esters of fatty acids having a carbon number of 14 or more other than mycolic acid with a mono- or disaccharide in an ELISA method for detecting an antibody against an acid-fast bacterial antigen in a serum sample.

2. The use as claimed in Claim 1 wherein said ester is a C₁-C₆ alkyl ester.

3. The use as claimed in Claim 1 wherein said ester is an ester with trehalose or glucose.

4. The use as claimed in Claim 1 wherein said ester is a mono- to tetra-substitution product of trehalose at a position selected from the 2, 2′, 3, 3′, 6 and 6′ positions.

5. The use as claimed in Claim 1 wherein said ester is at least one ester selected from the group consisting of 6,6′-di-O-palmitoyl-α,α-trehalose, 6,6′-di-O-mycoloyl-α,α-trehalose, 2,6′-di-O-oleoyl-α,α-trehalose, 6′-O-corynomycoloyl-2-O-pentadecanoyl-α,α-trehalose, 2-O-icosanoyl-6′-O-(3-hydroxy-2-n-octadecanyl-tetracosanoyl)-α,α-trehalose, 2-O-(9-octadecenoyl)-6′-O-(3-hydroxy-2-tetradecanyl-11-icosenoyl)-α,α-trehalose, 2-O-pentadecanoyl-3-O-octadecanoyl-6′-O-(3-hydroxy-2-n-tetradecanyl-docosanoyl)-α,α-trehalose, 2,3-di-O-eicosanoyl-6′-O-(3-hydroxy-2-n-octadecanyl-tetracosanoyl)-α,α-trehalose and 2,3-di-O-(9-octadecenoyl)-6′-O-(3-hydroxy-2-tetradecanyl-11-icosenoyl)-α,α-trehalose.

6. The use as claimed in any of claims 1 - 5 wherein the antibody against an acid-fast bacterial antigen in the sample is an antibody contained in a body fluid of a patient infected with tubercule bacillus or an acid-fast bacterium closely related thereto.

7. The use according to any of claims 1 - 6 for the diagnosis of acid-fast bacterial infections, which comprises determining the difference in optical density between a sample taken from a healthy human and a sample taken from the subject.

## Patentansprüche

1. Verwendung einer aus der aus Mykolsäuren, Mykolsäuresalzen, Mykolsäureestern und Estern von anderen Fettsäuren als Mykolsäure mit der Kohlenstoffzahl 14 oder größer bestehenden Gruppe ausgewählten Verbindung mit einem Mono- oder Disaccharid in einem ELISA-Verfahren zum Nachweisen eines Antikörpers gegen ein säurebeständiges bakterielles Antigen in einer Serumprobe.

2. Verwendung wie in Anspruch 1 beansprucht, wobei der Ester ein C₁-C₆-Alkylester ist.

3. Verwendung wie in Anspruch 1 beansprucht, wobei der Ester ein Ester mit Trehalose oder Glucose ist.

4. Verwendung wie in Anspruch 1 beansprucht, wobei der Ester ein Mono- oder Tetrasubstitutionsprodukt von Trehalose in einer aus der 2-, 2′-, 3-, 3′-, 6- und 6′-Stellung ausgewählten Stellung ist.

5. Verwendung wie in Anspruch 1 beansprucht, wobei der Ester wenigstens ein aus der aus 6,6′-Di-O-palmitoyl-α,α-trehalose, 6,6′-Di-O-mykolyol-α,α-trehalose, 2,6′-Di-O-oleoyl-α,α-trehalose, 6′-O-Corynomykoloyl-2-O-pentadecanoyl-α,α-trehalose, 2-O-Icosanoyl-6′-O-(3-hydroxy-2-n-octadecanyl-tetracosanoyl)-α,α-trehalose, 2-O-(9-Octadecenoyl)-6′-O-(3-hydroxy-2-tetradecanyl-11-icosenoyl)-α,α-trehalose, 2-O-Pentadecanoyl-3-O-octadecanoyl-6′-O-(3-hydroxy-2-n-tetradecanyl-docosanoyl)-α,α-trehalose, 2,3-Di-O-eicosanoyl-6′-O-(3-hydroxy-2-n-octadecanyl-tetracosanyl)-α,α-trehalose und 2,3-di-O-(9-octadecenoyl)-6′-O-(3-hydroxy-2-tetradecanyl-11-icosenoyl)-α,α-trehalose bestehenden Gruppe ausgewählter Ester ist.

6. Verwendung wie in einem der Ansprüche 1 - 5 beansprucht, wobei der Antikörper gegen ein säurebeständiges bakterielles Antigen in der Probe ein Antikörper ist, der in einer Körperflüssigkeit eines Patienten enthalten ist, der mit dem Tuberkelbazillus oder einem damit eng verwandten, säurebeständigen Bakterium infiziert ist.

7. Verwendung gemäß einem der Ansprüche 1 - 6 zur Diagnose von Infektionen mit säurebeständigen Bakterien, welche das Bestimmen des Unterschieds der optischen Dichte zwischen einer einem gesunden Menschen entnommenen Probe und einer dem Patienten entnommenen Probe umfaßt.

## Revendications

1. Utilisation d'un composé choisi dans le groupe comportant les acides mycoliques, les sels des acides mycoliques, les esters des acides mycoliques et les esters des acides gras ayant un nombre de carbone de 14 ou plus, autres que l'acide mycolique, avec un mono ou disaccharide dans une méthode ELISA pour détecter un anticorps contre un antigène de bactérie résistant aux acides dans un échantillon de sérum.

2. Utilisation telle que revendiquée dans la revendication 1, où ledit ester est un ester d'alkyle en C₁-C₆.

3. Utilisation telle que revendiquée dans la revendication 1, où ledit ester est un ester avec le tréhalose ou le glucose.

4. Utilisation telle que revendiquée dans la revendication 1, où ledit ester est un produit de mono à tétrasubstitution du tréhalose à une position choisie parmi les positions 2, 2′, 3, 3′, 6 et 6′.

5. Utilisation telle que revendiquée dans la revendication 1, où ledit ester est au moins un ester choisi dans le groupe formé par le
6,6′-di-O-palmitoyl-α,α -tréhalose,
le 6,6′-di-O-mycoloyl-α,α -tréhalose, le 2,6′-di-O-oléoyl-(α,α -tréhalose,
le 6′-O-corynomycoloyl-2-O-pentadécanoyl-α,α-tréhalose,
le 2-O-icosanoyl-6′-O-(3-hydroxy-2-n-octadécanyl-tétracosanoyl)-α,α -tréhalose,
le 2-O-(9-octadécénoyl)-6′-O-(3-hydroxy-2-tétradécanyl-11-icosénoyl)-α,α -tréhalose,
le 2-O-pentadécanoyl-3-O-octadécanoyl-6′-O-(3-hydroxy-2-n-tétradécanyl-docosanoyl)-α,α-tréhalose,
le 2,3-di-O-eicosanoyl-6′-O-(3-hydroxy-2-n-octadécanyl-tétracosanoyl)-α,α-tréhalose et
le 2,3-di-O-(9-octadécénoyl)-6′-O-(3-hydroxy-2-tétradécanyl-11-icosénoyl)-α,α- tréhalose.

6. Utilisation telle que revendiquée dans l'une quelconque des revendications 1 à 5, où l'anticorps contre un antigène de bactérie résistant aux acides dans l'échantillon est un anticorps contenu dans un fluide du corps d'un patient infecté par un bacille de tubercule ou une bactérie résistant aux acides qui lui est étroitement apparentée.

7. Utilisation telle que revendiquée dans l'une quelconque des revendications 1 à 6, pour le diagnostic d'infections par des bactéries résistant aux acides, qui comporte la détermination de la différence en densité optique entre un échantillon prélevé chez un humain en bonne santé et un échantillon prélevé chez le sujet.
